# EUROPÄISCHE PATENTSCHRIFT

(11) **EP 3 925 583 B1**
(45) Veröffentlichungstag und Bekanntmachung des Hinweises auf die Patenterteilung: **10.01.2024**
(21) Anmeldenummer: 21178577.9
(22) Anmeldetag: 09.06.2021
(51) Int. Cl.: A61F 9/008

(54) **VITREKTOMIEVORRICHTUNG MIT NADEL UND PULSLASER UND HERSTELLUNGSVERFHAREN**
VITRECTOMY DEVICE WITH NEEDLE AND PULSE LASER AND METHOD OF MANUFACTURING
DISPOSITIF DE VITRÉCTOMIE AVEC AIGUILLE ET LASER À IMPULSION ET PROCÉDÉ DE FABRICATION

(30) Priorität: 16.06.2020 DE 102020115885
(43) Veröffentlichungstag der Anmeldung: 22.12.2021
(73) Patentinhaber: A.R.C. Laser GmbH, 90411 Nürnberg (DE)
(72) Erfinder: Thyzel, Reinhardt, 90452 Eckental (DE)
(74) Vertreter: Schröer, Gernot H.

(56) Entgegenhaltungen:
- WO-A1-2009/036818
- DE-A1- 19 907 012
- DE-U1-202018 105 448
- US-A1- 2016 120 699

## Beschreibung

Die Erfindung betrifft eine Vitrektomienadel, ein Vitrektom, eine Vitrektomievorrichtung und ein Verfahren zur Herstellung einer Vitrektomienadel.

Bei der Vitrektomie wird der Glaskörper (Corpus Vitreum) des Auges mittels eines Instrumentes, das man Vitrektom oder Vitrektor nennt, sukzessive oder in kleinen Segmenten abgetragen (Ablation).

Die Vitrektomie wird insbesondere bei der Behandlung von Erkrankungen oder Ablösungen der Netzhaut (Retina) oder bei Blutungstrübungen eingesetzt. Es werden drei kleine Schnitte in der Lederhaut in einem *pars plana* genannten Teilbereich erzeugt als Zugänge für das Vitrektomie-Instrument, für eine Lichtquelle und für ein Infusions- und Absaugwerkzeug. In diese Zugänge werden kleine Zugangshülsen, sogenannte Trokare, eingesetzt, durch die die Instrumente in den Augapfel eingeführt werden.

Während der Operation wird der Druck in dem Augenhöhlenraum aufrechterhalten durch die Infusion einer überwiegend aus Wasser bestehenden Lösung. Der nach dem Abtragen des Glaskörpers entstandene Hohlraum wird nun zumeist mit einem Ersatzmaterial, zum Beispiel Silikonöl oder auch Gas oder Perfluorcarbonen, gefüllt, das einen entsprechenden Anlagedruck auf die Netzhaut ausübt, die dann beispielsweise durch Laserkoagulation wieder angeschweißt werden kann.

Die in der Praxis eingesetzten Vitrektome arbeiten mechanisch und umfassen kleine Klingen oder Schneiden oder Pinzetten oder Haken, die mittels eines Trägers innerhalb eines geraden Röhrchens vor- und zurückbewegt werden, wobei die derart abgetragenen oder abgeschnittenen Segmente des Glaskörpers dann mittels des Absauginstruments oder durch das Vitrektom selbst abgesaugt werden.

Aus WO 2015/135544 A1 ist ein Vitrektom bekannt, das zur Abtrennung von Glaskörpermaterial in vivo mechanische bewegte Schneidelemente umfasst.

Bekannte Vitrektome sind wegen des Schneidmechanismus meist nur gerade oder geradlinig ausgebildet. Ein gekrümmtes Vitrektom mit mechanischem Schneidmechanismus ist aus der US 2017/0071788 A1 bekannt.

Bei der Vitrektomie wird das Vitrektom durch einen Trokar durchgeführt und im Glaskörper Stück für Stück bewegt, bis der ganze Glaskörper oder ein abzutragender Teil abgetragen ist. Bei gerade ausgebildeten Vitrektomen muss hierbei ein ziemlich weiter Raumwinkel oder Schwenkbereich des Vitrektoms genutzt werden, um alle Bereiche des Glaskörpers zu erreichen. Dies führt zu erheblichen Kräften im Bereich des Trokars und des umgebenden Gewebes und kann zur Folge haben, dass der Eintrittsschnitt, durch den der Trokar hindurchgeführt ist, weiter aufreißt und genäht werden muss. Mechanische Vitrektome haben zusätzlich den Nachteil, dass die mechanischen Schneidbewegungen zu weiteren mechanischen Belastungen im Auge und im Bereich des Trokars führen können.

In DE 20 2018 105 448 U1 wird ein als Laserinstrument ausgebildetes Vitrektom vorgeschlagen. Es arbeitet mit einer Photoemulsifikation, ähnlich wie es bereits bei der Phako-Emulsifikation des Katarakts eingesetzt wird. Dieses Laserinstrument ist gerade ausgebildet und wird ebenso wie die gerade ausgebildeten Vitrektome mittels einer Schwenkbewegung in einem Trokar bewegt. Bei dem aus DE 20 2018 105 448 U1 bekannten Laserinstrument zur Vitrektomie liegt die Austrittsöffnung der in das Auge einzuführenden Hohlnadel in einem seitlichen Bereich des Endbereichs der Hohlnadel. Der Abstand dieser Öffnung von der Zentralachse der Hohlnadel entspricht dem halben Durchmesser der Hohlnadel des Laserinstruments in einem Bereich von 0,175 bis 0,375 mm.

Die US 2016/0120699 A1 beschreibt ein Vitrektom mit einer zur Einführung in das Auge ausgebildeten Nadel, die gekrümmt sein kann. Bei der US'699 ist ein Lichtleiter in der Nadel angeordnet, der bzw. dessen freies Ende im Betrieb in der Nadel bewegt wird, so dass in die Nadel eingetretenes Glaskörpermaterial durch Laserlicht, das durch den Lichtleiter austritt, unmittelbar zerschnitten wird.

Die WO 2009/036818 offenbart ein Vitrektom mit einer mechanisch drehbaren, prismen- oder sternförmig ausgebildeten Schneideinrichtung zur Zertrümmerung von Glaskörpermaterial. Zu Zwecken der Beleuchtung des Operationsbereichs ist in der Vitrektomienadel ein Lichtleiter angeordnet.

Die DE19907012 offenbart ein Vitrektom mit einer Anordnung von reflektierenden Flächen in der Nähe der axialen Öffnung der Hohlnadel, wobei durch eine seitliche Auskopplung des Laserlichts aus einem Lichtleiter bei gleichbleibender oder verkleinerter Laserenergie die Auskopplung der Schockwellen am Ausgang der Hohlnadel erzeugt wird.

Bei der Phako-Emulsifikation besteht überhaupt kein Problem, alle Bereiche der vergleichsweise sehr kleinen Linse mit dem geraden Laserinstrument zu erreichen. Hier besteht auch keine Gefahr einer Verletzung oder eines Ausreißens des Eintrittsschnitts in der Hornhaut bei der Operation, weil die Schwenkbewegung des Instruments aufgrund der geringen Größe der Linse sehr klein ist. Bei der Vitrektomie entstehen dagegen aufgrund der viel größeren Schwenkbewegung wesentliche grö-ßere Auslenkungen durch das Vitrektom im Bereich des Schnittes und es kommt deshalb häufig zu den schon beschriebenen Risswunden, die genäht werden müssen.

Es ist daher Aufgabe der Erfindung, eine Vitrektomievorrichtung und ein Verfahren zur Herstellung einer Vitrektomievorrichtung bereitzustellen, mit denen die Nachteile des Stands der Technik beseitigt werden können. Insbesondere sollen eine Vitrektomievorrichtung angegeben werden, die eine für das zu behandelnde Auge vergleichsweise schonende Entfernung des Glaskörpers ermöglichen. Ferner sollen insbesondere eine Vitrektomievorrichtung angegeben werden, die eine besonders effiziente Entfernung des Glaskörpers ermöglichen und für den anwendenden Arzt oder Bediener eine vereinfachte, insbesondere ergonomisch verbesserte Handhabung, bei der Durchführung einer Vitrektomie bieten. Ferner soll ein Verfahren zur Herstellung einer entsprechenden Vitrektomievorrichtung bereitgestellt werden.

Diese Aufgabe wird gelöst durch die unabhängigen Patentansprüche. Ausgestaltungen ergeben sich aus den abhängigen Patentansprüchen sowie aus der nachfolgenden Beschreibung.

Nach einer Ausgestaltung ist eine Vitrektomienadel vorgesehen. Die Vitrektomienadel umfasst eine Hohlnadel, beispielsweise in Form einer Kanüle oder eines dünnen, dünnwandigen rohrförmigen Hohlkörpers.

Die Hohlnadel umfasst ein distales Ende zum Einführen in den Glaskörper eines Auges zur Durchführung einer Vitrektomie. Die Vitrektomienadel ist insbesondere derart ausgebildet, dass diese mit dem distalen Ende durch ein Trokar ins Auge und in den Glaskörper zur Durchführung einer Vitrektomie eingeführt werden kann.

Ferner umfasst die Vitrektomienadel einen in der Hohlnadel zu dem distalen Ende geführten Laserlichtleiter. Der Laserlichtleiter ist in der Hohlnadel geführt und darin in geeigneter Weise festgelegt. Insbesondere kann der Laserlichtleiter durch eine oder mehrere Klebebefestigungen befestigt sein. Der Laserlichtleiter kann beispielsweise einen Außendurchmesser von 250 µm bis 300 µm, insbesondere von etwa 270 µm, aufweisen. Der Durchmesser des Laserlichtleiters kann abhängig vom Innendurchmesser und/oder Außendurchmesser der Hohlnadel gewählt sein, wobei der Durchmesser des Laserlichtleiters vorzugsweise umso größer ist, je größer der Innen- oder Außendurchmesser der Hohlnadel ist. Beispielsweise kann bei einer 23G Hohlnadel (0,6 mm Außendurchmesser) der Laserlichtleiter einen Außendurchmesser von etwa 270 µm aufweisen.

Der Laserlichtleiter weist eine zum distalen Ende hin orientierte Lichtaustrittsfläche auf, wobei im Bereich der Lichtaustrittsfläche und der axial nach innen gewandeten Fläche des distalen Endes, insbesondere zwischen der Lichtaustrittsfläche und der nach innen gewandten Fläche des distalen Endes, in der Hohlnadel eine Kavität ausgebildet ist. Der Laserlichtleiter kann beispielsweise derart ausgebildet, eingerichtet und positioniert sein, dass die Lichtaustrittsfläche des Laserlichtleiters von der axialen Innenwandung am geschlossenen Ende des distalen Endes 0,9 mm bis 1,2 mm beabstandet ist. Vorzugsweise ist der Abstand zwischen der Lichtaustrittsfläche abhängig vom Außendurchmesser der Hohlnadel, und umso kleiner je kleiner der Außendurchmesser ist.

Die Wandung der Hohlnadel weist im Bereich der Kavität einen bezüglich der Mittelachse der Hohlnadel radial verlaufenden Durchbruch, insbesondere eine Austrittsöffnung, auf.

Die Hohlnadel weist am proximalen Ende, d.h. an dem vom distalen Enden entfernt gelegenen Ende, einen geraden Abschnitt auf. Das proximale Ende der Hohlnadel kann beispielsweise dazu ausgebildet sein, an oder mit einem Handstück oder Griffstück befestigt zu werden. Der gerade Abschnitt kann beispielsweise derart ausgebildet sein, dass dieser bei der Durchführung einer Vitrektomie am Auge in einem Trokar gelegen ist bzw. positioniert werden kann.

Die Mittelachse der Hohlnadel definiert im Bereich des geraden Abschnitts des proximalen Endes eine Zentralachse.

Bei der Vitrektomienadel ist nun vorgesehen, dass die Mittelachse der Hohlnadel im Bereich des Durchbruchs bzw. der Kavität um einen vorgegebenen radialen Mindestabstand von der Zentralachse beabstandet ist. Der vorgegebene Mindestabstand kann, wie weiter unten genauer beschrieben ist, beispielsweise dadurch erreicht werden, dass die Hohlnadel gekrümmt oder gebogen ist. Möglich ist auch, dass die Vitrektomienadel ausgehend von dem geraden Abschnitt zumindest abschnittsweise schräg, insbesondere unter einem Spitzen Winkel, zur Zentralachse verläuft.

Durch den vorgegebenen radialen Mindestabstand können Kräfte, die bei der Durchführung einer Vitrektomie im Bereich des Eintrittschnitts der Vitrektomienadel in das Auge wirken, und mithin die Gefahr eines Aufreißens des Eintrittschnitts bzw. der damit verbundenen Wunde reduzieren werden. Insbesondere kann dadurch, dass der Durchbruch, d.h. der durch den Durchbruch definierte Arbeitsbereich, der Vitrektomienadel gegenüber der Zentralachse radial weiter nach außen versetzt ist erreicht werden, dass ein wesentlich größerer Raumwinkelbereich allein schon durch Drehung der Vitrektomienadel um die Zentralachse möglich ist. Schwenkbewegungen, mit denen auf den Eintrittsschnitt wirkende Kräfte erzeugt werden sind mithin nur noch in kleinem Umfang erforderlich, oder können u.U. sogar ganz vermieden werden.

Zwar erfordert die Einführung der Vitrektomienadel bzw. eines entsprechenden Laserinstruments in das Auge, beispielsweise durch ein Trokar, größere Beachtung, da eine etwas kompliziertere Bewegung erforderlich ist verglichen mit einem durchgehend geradlinigen Instrument. Jedoch überwiegen die nach dem Einführen der Vitrektomienadel in das Auge erreichten Vorteile, wie z.B. die deutlich vereinfachte Handhabung und der deutlich reduzierte Krafteintrag in den Eintrittsschnitt bei der Durchführung der Vitrektomie deutlich.

Um die Einführung der Vitrektomienadel zu vereinfachen und zu erleichtern, kann die Vitrektomienadel, wie nachfolgend noch genauer ausgeführt wird, speziell geformt sein. Beispielsweise kann die Vitrektomienadel gekrümmt ausgeführt sein, so dass eine stetige und insbesondere kantenfreie Form der Vitrektomienadel eine entsprechend glatte und stetige Einführbewegung ermöglicht. Derartige Bewegungen sind insbesondere auch hinsichtlich einer Reduktion der beim Einführen auf den Eintrittsschnitt ggf. wirkenden Kräfte von Vorteil.

Bei der Durchführung einer Vitrektomie wird die Vitrektomienadel durch einen Eintrittsschnitt im Bereich des Auges, beispielsweise unter Verwendung eines Trokars, in das Auge eingeführt, so dass das distale Ende mit dem Durchbruch im Glaskörper des Auges positioniert ist. Sodann wird der Laserlichtleiter durch eine mit diesem gekoppelte Laserlichtquelle mit Laserlicht, mit Laserpulsen, beaufschlagt. Beispielsweise kann als Laserlichtquelle ein Nd:YAG Pulslaser verwendet werden. Die Laserpulse ist mit einer Pulsfrequenz von 40 Hz bis 60 Hz und einer Pulsenergie im Bereich von 0,5 bis 3 mJ, insbesondere im Bereich von 1 bis 2 mJ, erzeugt.

Die im inneren der Vitrektomienadel an der Lichtaustrittsfläche des Laserlichtleiters ausgehenden Laserpulse erzeugen im Bereich der Kavität und des Durchbruchs Schockwellen, die durch den Durchbruch austreten und entsprechend das Glaskörpermaterial zertrümmern, also zersetzen. Mithin wird das Glaskörpermaterial photolysisch abgebaut bzw. zersetzt. Das abgebaute bzw. zersetzte Glaskörpermaterial kann mittels einer geeigneten Absaugeinrichtung abgesaugt werden (Aspiration), die beispielsweise in einem Handstück oder einem Griffstück integriert oder als gesondertes Instrument ausgebildet sei kann. Zur Aufrechterhaltung des Augeninnendrucks wird bei der Vitrektomie das entnommene Glaskörpermaterial üblicherweise mit Gas oder einer Flüssigkeit, insbesondere Silikonöl ersetzt (Irrigation).

Nach Ausgestaltungen kann die Hohlnadel ausgehend von dem geraden Abschnitt am proximalen Ende zum distalen Ende hin eine Krümmung, insbesondere einen gekrümmten Verlauf, beispielsweise mit zumindest einer Biegung, mit einem, vorzugsweise konstanten, Krümmungsradius aufweisen. Wie bereits erwähnt, können derart geformte Vitrektomienadeln trotz der Krümmung oder gekrümmten Verlaufs vergleichsweise einfach, beispielsweise durch einen im Wesentlichen kontinuierlich erfolgenden Bewegungsablauf, durch den Eintrittsschnitt, insbesondere ein Trokar, in das Auge eingeführt werden. Unnötige Krafteinwirkungen auf den Eintrittsschnitt können zumindest weitgehend vermieden werden.

Nach Ausgestaltungen erstreckt sich die Krümmung bzw. der gekrümmte Verlauf ausgehend vom geraden Abschnitt im Wesentlichen bis zum distalen Ende hin. In Ausgestaltungen kann es sich bei der Krümmung, ausgehend vom geraden Abschnitt zum distalen Ende hin, um eine kontinuierliche Krümmung handeln.

Damit kann insbesondere eine kontinuierliche Bewegungsführung beim Einführen der Vitrektomienadel erreicht werden. Ferner kann bei geeigneter Krümmung ein für das Entfernen des Glaskörpers vorteilhafter Aktionsradius bereits durch Drehen der Vitrektomienadel um die Zentralachse erreicht werden, so dass die Zahl der erforderlichen Verkippungen oder Verschwenkungen der Vitrektomienadel deutlich reduziert werden kann. Die genannten Vorteile gelten entsprechend für die vorweg und nachfolgend beschriebenen Geometrien und Formen der Vitrektomienadel.

Nach Ausgestaltungen kann die Hohlnadel ausgehend von der Krümmung zum distalen Ende hin zumindest einen weiteren geraden Abschnitt aufweisen. In diesem Falle liegt entsprechend keine kontinuierliche Krümmung vor.

Beispielsweise kann sich an den vom proximalen Ende ausgehenden geraden Abschnitt eine Krümmung anschließen, an die sich zum distalen Ende hin ein weiterer gerader Abschnitt anschließt. Auch solche Geometrien und Formen ermöglichen trotz der Abweichung von einer gänzlich geraden Form ein vergleichsweise einfaches Einführen der Vitrektomienadel bei gleichzeitig vorteilhaftem Aktionsradius des Arbeitsbereichs, sprich des Durchbruchs an der Hohlnadel.

Nach Ausgestaltungen weist die Hohlnadel einen Außendurchmesser von 0,55 mm bis 0,65 mm, vorzugsweise 0,6 mm auf. Nach weiteren Ausgestaltungen kann die Hohlnadel einen Innendurchmesser von 0,35 mm bis 0,55 mm, insbesondere von 0,35 mm bis 0,45 mm, vorzugsweise von etwa 0,4 mm aufweisen. In weiteren Ausgestaltungen kann die Hohlnadel eine längs der Mittelachse gemessene Länge von 18 mm bis 30 mm, insbesondere zwischen 20 mm und 27 mm, vorzugsweise etwa 25 mm aufweisen. Solche Vitrektomienadeln ermöglichen insbesondere minimalinvasive Eingriffe im Zusammenhang mit der Entfernung des Glaskörpers oder von Teilen davon, beispielsweise bei Eintrittsschnitten im Bereich des *pars plana.*

Nach Ausgestaltungen liegt der radiale Mindestabstand im Bereich von mindestens 5 mm bis 30 mm. Insbesondere liegt es im Rahmen der Erfindung, dass Vitrektomienadeln gemäß den beschriebenen Geometrien und Formen mit den genannten radialen Mindestabständen hergestellt werden können. Mithin können Vitrektomienadeln mit unterschiedlichen Aktionsradien bezüglich einer Drehung um die Zentralachse für unterschiedliche Anwendungen und/oder unterschiedliche Augengrößen hergestellt werden.

Nach Ausgestaltungen kann der Durchbruch an einer der Zentralachse zugewandten Seite der Hohlnadel oder an einer der Zentralachse abgewandten Seite der Hohlnadel ausgebildet sein. Durch diese beiden Varianten kann insbesondere die Anwendungsspezifität der Vitrektomienadel vorteilhafter Weise angepasst werden. Beispielsweise können Vitrektomienadeln mit unterschiedlich gelegenen Durchbrüchen bereitgestellt werden, die jeweils in besonderer Weise zum Entfernen gewisser Bereiche des Glaskörpers geeignet sind. Denkbar ist beispielsweise, dass zum Entfernen von mittig im Glaskörper gelegenen Bereichen Vitrektomienadeln mit Durchgangsöffnungen verwendet werden, die von der Zentralachse abgewandt, beispielsweise an einem konkav gekrümmten Abschnitt der Hohlnadel, gelegen sind, und dass zum Entfernen von randseitig des Glaskörpers gelegenen Bereichen Vitrektomienadeln mit Durchgangsöffnungen verwendet werden, die der Zentralachse zugewandt, beispielsweise an einem konvex gekrümmten Abschnitt der Hohlnadel, gelegen sind. Jedoch eignen sich beide Ausführungsformen gleichermaßen auch für die Entfernung des gesamten Glaskörpers, wobei sich ggf. Art und Umfang der erforderlichen Schwenkbewegungen unterscheiden.

Nach einer Ausgestaltung ist ein Vitrektom, d.h. ein chirurgisches Instrument zur Durchführung einer Vitrektomie, vorgesehen, wobei das Vitrektom ein Handstück und eine mit dem Handstück verbundene Vitrektomienadel nach einem der hierin beschriebenen Ausgestaltungen umfasst. Vorteile und vorteilhafte Wirkungen der im Zusammenhang mit der beschriebenen Vitrektomienadel gelten entsprechend für das Vitrektom.

Nach Ausgestaltungen kann die Vitrektomienadel lösbar am Handstück befestigt sein, insbesondere mittels eines am proximalen Ende der Vitrektomienadel ausgebildeten Schraubverbinders, der z.B. eingerichtet ist zur Verschraubung mit dem Handstück. In Ausgestaltungen kann die Vitrektomienadel fest mit dem Handstück verbunden sein, beispielsweise mittels einer Klebeverbindung.

Nach Ausgestaltungen ist eine Vitrektomievorrichtung vorgesehen, die eine Laserlichtquelle, eine Steuereinrichtung zur Steuerung der Laserlichtquelle und eine Anschlussschnittstelle zum Anschließen eins Vitrektoms nach einer der hierin beschriebenen Ausgestaltungen umfasst. An der Anschlussschnittstelle kann ein Vitrektom angeschlossen sein. Die die Steuereinrichtung ist dazu eingerichtet, bei angeschlossenem Vitrektom die Laserlichtquelle zur Ausführung bzw. Durchführung einer Vitrektomie anzusteuern. Beispielsweise kann die Steuereinrichtung die Laserlichtquelle derart ansteuern, beispielsweise gemäß einer über eine Benutzerschnittstelle erfolgte Aktivierung, dass diese zur Durchführung einer Vitrektomie geeignete Laserlichtpulse abgibt, die dann über die Virtrektomienadel appliziert werden können.

Die Vitrektomievorrichtung kann weitere Komponenten umfassen, die zur Durchführung einer Vitrektomie erforderlich sind, wie beispielsweise eine Aspirationsvorrichtung zum Absaugen von zertrümmertem Glaskörpermaterial, sowie eine Irrigationsvorrichtung zum Einbringen von Spülflüssigkeit usw.

Nach einer Ausgestaltung ist ferner ein Verfahren zur Herstellung einer Vitrektomienadel.

Das Verfahren umfasst die folgenden Schritte:
- Bereitstellen eines langgestreckten massiven, insbesondere zylindrischen, Rohlings,
- Erzeugen eines Hohlkörpers aus dem Rohling durch Bohren eines zentralen, in Längsrichtung des Rohlings verlaufenden Sacklochs mittels eines Bohrwerkzeugs;
- Erzeugen eines bezüglich der Längsrichtung radialen Durchbruchs an dem geschlossenen Ende des Sacklochs mittels eines Fräswerkzeugs derart, dass der Durchbruch radial in das Sackloch mündet;
- Erwärmen des Hohlkörpers auf eine Umformungstemperatur, im Allgemeinen insbesondere im Bereich zwischen 60°C und 140°C, vorzugsweise im Bereich zwischen 100°C und 130°C, weiter vorzugsweise bei ungefähr 120°C; und
- Umformen des Hohlkörpers auf die finale Endgeometrie der Vitrektomienadel mittels eines Umformwerkzeugs.

Vorzugsweise weist der zur Herstellung der Vitrektomienadel verwendete Rohling einen Außendurchmesser von 0,2 mm bis 1 mm auf.

Der radiale Durchbruch wird vorteilhafter Weise vor der Umformung, d.h. vor dem Umformschritt, erzeugt.

Die Verwendung eines Sacklochs, d.h. einer axialen Bohrung, die sich nicht durch den ganzen Rohling hindurch erstreckt, hat insbesondere den Vorteil, dass der Hohlkörper am distalen, axialen Ende bereits verschlossen ist, und insoweit ein gesonderter Verfahrensschritt zum Verschließen des distalen Endes nicht erforderlich ist.

Das oder die zur Umformung der Vitrektomienadel verwendeten Umformwerkzeuge, beispielsweise Gesenke, können beispielsweise eingerichtet sein zur Formung der Außenkontur einerseits und zur Formung der Innenkontur andererseits.

Der Hohlkörper kann beispielsweise resistiv elektrisch (ohmsch) und/oder induktiv erwärmt werden.

Beispielhafte Ausgestaltungen der Erfindung werden nachfolgend anhand der anliegenden Figuren beschrieben. Es zeigen
- FIG. 1: eine Vitrektomienadel nach einer ersten Ausgestaltung im Rahmen der Erfindung;
- FIG. 2: eine Vitrektomienadel nach einer zweiten Ausgestaltung im Rahmen der Erfindung;
- FIG. 3: eine vergrößerte Darstellung einer Vitrektomienadel mit beispielhaften Abmessungen;
- FIG. 4 und 5: zwei weitere Ausgestaltungsvarianten einer Vitrektomienadel;
- FIG. 6: schematisch eine Vitrektomievorrichtung mit Vitrektom;
- FIG. 7: schematisch und nicht maßstabsgetreu eine Anwendung der Vitrektomienadel;
- FIG. 8: ein weiteres Ausführungsbeispiel eines Vitrektoms;
- FIG. 9: einen vergrößerten Abschnitt des distalen Endes der Vitrektomienadel des Vitrektoms der FIG. 8; und
- FIG. 10: Verfahrensschritte eines Verfahrens zur Herstellung der Vitrektomienadel.

Sofern nicht anderweitig explizit beschrieben, werden gleiche oder funktionsgleiche Elemente in den Figuren mit den gleichen Bezugszeichen bezeichnet. Ferner sind die Figuren nicht zwingend maßstabsgetreu.

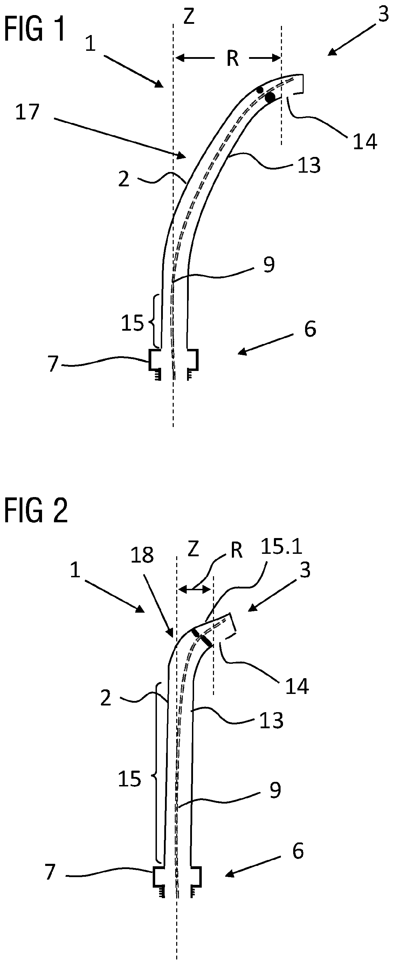

FIG. 1 zeigt eine Vitrektomienadel 1 nach einer ersten Ausgestaltung im Rahmen der Erfindung.

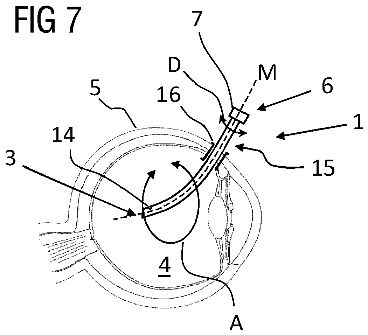

Die Vitrektomienadel 1 umfasst eine Hohlnadel 2 mit einem distalen Ende 3 zum Einführen in den Glaskörper 4 eines Auges 5 zur Durchführung einer Vitrektomie, was insbesondere auch FIG. 7 dargestellt ist.

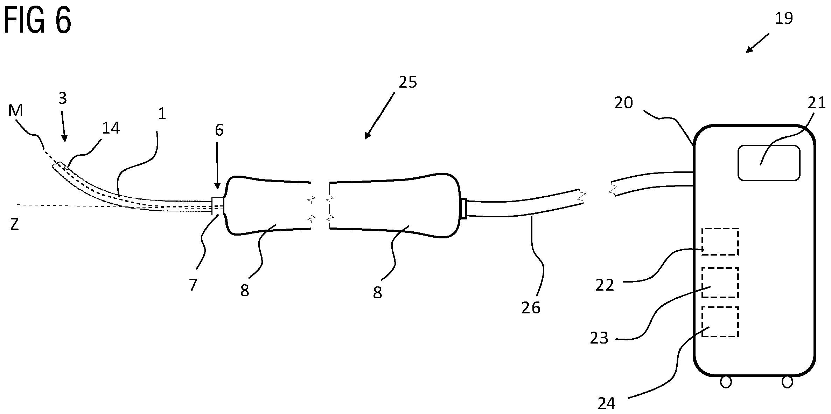

Die in den Figuren gezeigte Vitrektomienadel 1 umfasst beispielhaft an dem vom distalen Ende 3 abgewandten proximalen Ende 6 der Vitrektomienadel 1 einen Schraubverbinder 7 zur lösbaren Befestigung an einem in FIG. 6 dargestellten Griffteil 8. Zur Befestigung der Vitrektomienadel an einem Griffteil und/oder einer Halterung kommen alternativ oder ergänzend auch andere Befestigungsmöglichkeiten, wie insbesondere Klebeverbindeungen, in Betracht. Insbesondere kann die Vitrektomienadel auf- oder angeklebt sein.

Die Vitrektomienadel 1 umfasst ferner einen in der Hohlnadel 2 zu dem distalen Ende 3 geführten, und dort mittels geeigneter Befestigungselemente befestigten Laserlichtleiter 9 mit einer zum distalen Ende 3 hin orientierten Lichtaustrittsfläche 10.

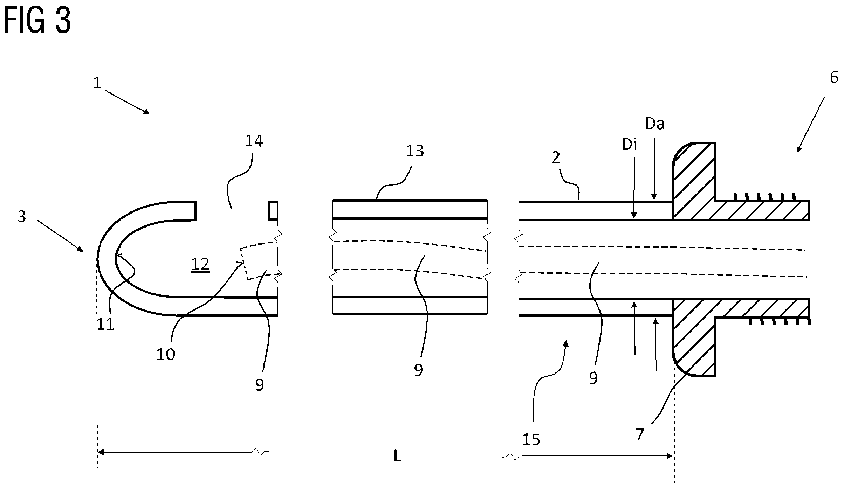

Wie insbesondere aus FIG. 3 ersichtlich ist, ist zwischen der Lichtaustrittsfläche 10 und der axial nach innen gewandeten Fläche 11 des distalen Endes 3 eine Kavität 12 ausgebildet. Ferner weist die Wandung 13 der Hohlnadel 2 im Bereich der Kavität 12 einen bezüglich der, aus Gründen der Übersichtlichkeit lediglich in FIG. 6 und FIG. 7 dargestellten, Mittelachse M der Hohlnadel 2 radial verlaufenden Durchbruch 14 auf. Dabei soll unter der Mittelachse M der Hohlnadel 2, bzw. der Vitrektomienadel 1, diejenige Achse verstanden werden, die der Form der Hohlnadel 2 bzw. Vitrektomienadel in deren Längsrichtung folgend mittig durch die Hohlnadel 2 bzw. die Vitrektomienadel 1 verläuft.

Am proximalen Ende 6 weist die Hohlnadel 2, insbesondere die Vitrektomienadel 1, einen geraden Abschnitt 15 auf. Bei dem geraden Abschnitt kann es sich beispielsweise um ein Anschlussstück, wie beispielsweise einen Schraubverbinder 7, zum Anschluss an ein Griffteil 8 (FIG. 6) handeln. Bei dem geraden Abschnitt 15 kann es sich jedoch auch, wie in den Figuren dargestellt, um einen Abschnitt der Hohlnadel 2 handeln.

Wie aus den Figuren ersichtlich ist, kann der gerade Abschnitt 15 bei unterschiedlichen Ausgestaltungen der Hohlnadel 2 unterschiedlich lange sein. In den gezeigten Ausgestaltungen weist der gerade Abschnitt 15 der Hohlnadel 2 eine Mindestlänge auf, die zumindest der axialen Länge eines in FIG. 7 schematisch gezeigten Trokars 16, die beispielsweise im Bereich von 20 nm bis 30 nm liegen kann, entspricht, durch den hindurch die Hohlnadel 2 in das Auge 5 bei einer Vitrektomie eingeführt wird. Mit dem geraden Abschnitt 15 kann die Hohlnadel 2 nach Durchführen durch den Trokar 16 um die Zentralachse Z der Vitrektomienadel 1 bzw. der Hohlnadel 2 rotiert werden, ohne dass Kippmomente auf den Trokar 16 ausgeübt werden.

Die Zentralachse Z ist im Rahmen der Erfindung als diejenige Achse zu verstehen, welche definiert ist durch, bzw. parallel verläuft durch die Mittelachse M im geraden Abschnitt 15 des proximalen Endes 6 der Hohlnadel 2, bzw. der Vitrektomienadel 1. Insbesondere ist die Zentralachse Z als diejenige Achse zu verstehen, welche durch die zur Mittelachse M im geraden Abschnitt 15 am proximalen Ende 6 kollinear verlaufende Gerade definiert ist.

Bei den Vitrektomienadeln 1 der Ausführungsbeispiele nach Figuren 1 bis 7 ist die Mittelachse M der Hohlnadel 2 bzw. der Vitrektomienadel 1 im Bereich des Durchbruchs 14 um einen vorgegebenen radialen Mindestabstand R von der Zentralachse Z beabstandet. Der radiale Mindestabstand R ist in den Figuren bezüglich des dem proximalen Endes zugewandten Rands des Durchbruchs 14 gezeigt. Möglich ist auch, dass für den radialen Mindestabstand R der Mittelpunkt der Öffnungsfläche des Durchbruchs 14 betrachtet wird. Unter dem Begriff eines vorgegebenen radialen Mindestabstands soll insbesondere verstanden werden, dass es sich bei dem Mindestabstand nicht um eine zufällige Abweichung der Zentralachse Z von der Mittelachse M handelt, sondern um einen bei der Herstellung der Vitrektomienadel 1 bzw. der Hohlnadel 2 konkret und vorgegeben eingestellten Abstand.

Wie weiter oben bereits erwähnt und diskutiert wurde, bietet die beschriebene Abweichung der Mittelachse M von der Zentralachse Z Vorteile hinsichtlich der Handhabung der Vitrektomienadel 1 sowie hinsichtlich der Krafteinwirkung auf den Eintrittsschnitt am Auge 5.

Die in FIG. 1 gezeigte Vitrektomienadel 1 weist ausgehend vom geraden Abschnitt 15 zum distalen Ende 3 hin eine kontinuierliche Krümmung 17 auf, wobei der Krümmungsradius längs der Mittelachse M variiert.

Im Vergleich zu der Vitrektomienadel 1 der FIG. 1 ist der gerade Abschnitt 15 der Hohlnadel 2 der Ausgestaltung nach FIG. 2 verlängert. Nach dem geraden Abschnitt 15 der Hohlnadel 2 der FIG. 2 folgt eine Krümmung 18, der wiederum ein, wenn auch kurzer, weiterer geradliniger Abschnitt 15.1 folgt.

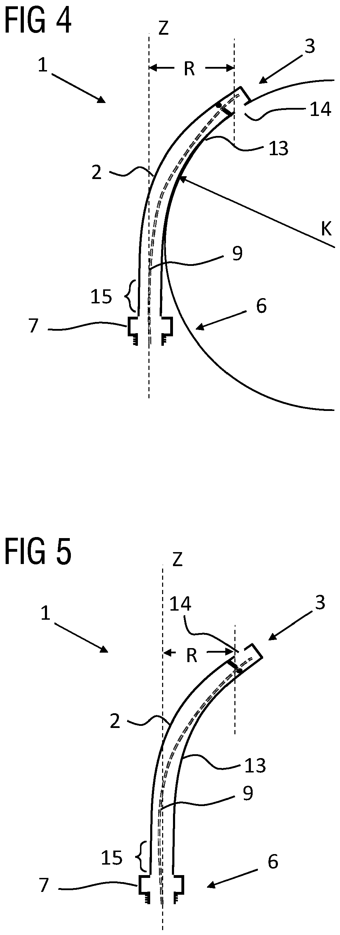

In der Ausgestaltung nach FIG. 4 bis 7 weist die Hohlnadel 2 ausgehend von dem geraden Abschnitt 15 zum distalen Ende 3 hin eine Krümmung mit kontinuierlicher Krümmung auf, wobei des Weiteren ein im Wesentlichen konstanter Krümmungsradius K umgesetzt ist. Der Übersichtlichkeit halber ist der Krümmungsradius K lediglich in FIG. 4 gezeigt.

Die Ausgestaltungen nach FIG. 4 und FIG. 5 unterscheiden sich lediglich in der Position des Durchbruchs 14 relativ zur Zentralachse Z. Während bei der FIG. 4 der Durchbruch 14 auf einer der Zentralachse Z abgewandten Seite der Hohlnadel 2 vorgesehen ist, ist bei der FIG. 5 der Durchbruch 14 auf der der Zentralachse Z zugewandten Seite der Hohlnadel 2 vorgesehen.

Beispielhafte Abmessungen der Vitrektomienadel 1 bzw. der Hohlnadel 2 sind in FIG. 3 angegeben. Entsprechend der FIG. 3 kann die Hohlnadel 2 einen Außendurchmesser Da von 0,4 mm aufweisen. Ein Innendurchmesser Di der Hohlnadel 2 kann dabei 0,25 mm betragen. Entlang der Mittelachse M gemessen, d.h. der Form der Hohlnadel 2 folgend, kann die Hohlnadel 2 eine Länge L von beispielsweise 25 mm aufweisen.

Eine Wandstärke der Vitrektomienadel kann beispielsweise bei etwa 0,1 mm liegen.

Gestützt auf die medizinische Fachterminologie kann die Größe der Vitrektomienadel im Bereich von 20G bis 27G liegen (G: Gauge). Diese Größen entsprechen Außendurchmessern im Bereich zwischen 0,4 mm (bei 27G) und 0,9 mm (bei 20G).

Der mit Bezug zu Figuren 1, 2, 3 und 4 gezeigte radiale Mindestabstand kann im Bereich von mindestens 5 mm bis 30 mm liegen.

FIG. 6 zeigt eine Vitrektomievorrichtung 19 umfassend eine fahrbar ausgebildete Zentraleinheit 20 mit Bedienkonsole 21, einer Laserlichtquelle 22, einer Aspirations- und Irrigationsvorrichtung 23 und einer Steuereinheit 24, die dazu eingerichtet ist, bei angeschlossenem Vitrektom 25 die Laserlichtquelle 22, die Aspirations- und Irrigationsvorrichtung 23 und ggf. andere Komponenten und Einheiten zur Ausführung einer Vitrektomie anzusteuern. Dazu kann die Steuereinheit 24 einen Speicher mit darauf gespeicherten computerlesbaren Befehlen umfassen, die bei Ausführung durch einen Prozessor die entsprechenden Einheiten zur Durchführung einer Vitrektomie ansteuern können, insbesondere gemäß einer Benutzereingabe über die Bedienkonsole 21 und/oder über das Griffstück 8 des Vitrektoms 25.

Das Vitrektom 25 umfasst die bzw. eine Vitrektomienadel 1, und das mit der Vitrektomienadel 1 verbundene Griffteil 8. Beispielsweise kann die Vitrektomienadel 1 mit dem Griffteil durch eine Klebeverbindung fest verbunden sein. Andere Verbindungsarten sind, wie weiter oben beschrieben, ebenfalls oder alternativ möglich. Durch das Griffteil 8 hindurch und mit diesem verbunden ist eine Steuer- und/oder Signalleitung 26, im Folgenden kurz Leitung, die wiederum mit der Zentraleinheit 20 verbunden ist. Die Leitung 26 ist zumindest dazu ausgebildet, Laserlicht, insbesondere Laserpulse, von der Laserlichtquelle 22 zum Laserlichtleiter 9 der Vitrektomienadel 1 zu übertragen. Beispielsweise kann in der Leitung 26 selbst ein (nicht gezeigter) Laserlichtleiter vorhanden sein, der bei angeschlossener Vitrektomienadel 1 mit dem Laserlichtleiter 9 gekoppelt ist.

Im Betrieb der Vitrektomievorrichtung 19 werden durch die Laserlichtquelle 22 beispielsweise Laserpulse erzeugt, und durch die Leitung 26 in den Laserlichtleiter 9 der Vitrektomienadel 1 eingekoppelt. An der Lichtaustrittsfläche 10 austretende Laserpulse führen zu einer photolysischen Zersetzung des Glaskörpermaterials, durch laserinduzierte Druckpulse, die durch Laserabsorption in der Kavität 12 erzeugt werden und durch den Durchbruch 14 hindurch das Glaskörpermaterial beaufschlagen. Das zertrümmerte oder zerstückelte Glaskörpermaterial kann mit der Aspirationseinheit abgesaugt werden, wobei mittels der Irrigationsvorrichtung Spülflüssigkeit und andere Medien eingebracht werden können.

Wie insbesondere aus der FIG. 7 ersichtlich ist, weist eine Vitrektomienadel 1 auf Grund der besonderen Form, insbesondere aufgrund des radialen Abstands des Durchbruchs 14 von der Zentralachse Z schon allen bei Ausführen einer Drehung D um die Zentralachse Z einen vergleichsweise großen Aktionsradius A auf. Aufgrund des vergleichsweise großen Aktionsradius A können bei Durchführung einer Vitrektomie bezüglich der Zentralachse Z ausgeführte Schwenkbewegen, durch die über den Trokar 16 Kräfte auf den Eingriffsschnitt im Auge ausgeübt werden können, auf ein Minimum reduziert werden, verbunden mit den weiter oben genannten Vorteilen.

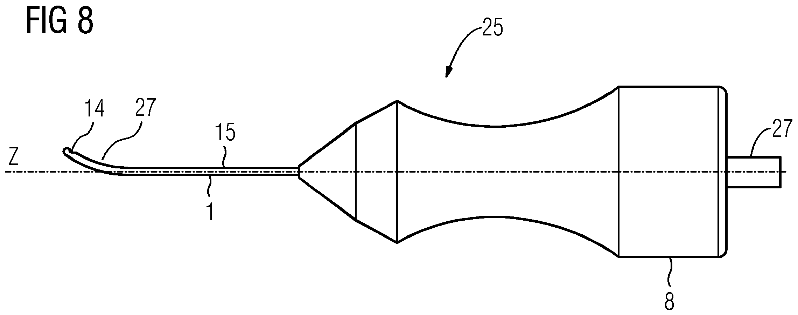

FIG. 8 zeigt ein weiteres Ausführungsbeispiel eines Vitrektoms 25. Das Vitrektom 25 der FIG. 8 umfasst ein Griffteil 8 mit einer Anschlussleitung bzw. einem Anschluss 27 zum Anschließen beispielsweise an eine wie im Zusammenhang mit FIG. 6 schematisch beschriebene Zentraleinheit 20.

Das Griffteil 8 kann bezüglich der Zentralachse Z rotationssymmetrisch ausgebildet sein. In Längsrichtung betrachtet etwa mittig umfasst das Griffteil 8 eine umlaufende konvexe Einbuchtung oder Ausnehmung, die zum Haltern des Vitrektoms 25 mit zwei oder mehr Fingern, insbesondere nach ergonomischen Gesichtspunkten, ausgebildet ist. In Ausgestaltungen kann das Griffteil auch eine von einer rotationssymmetrischen Form verschiedene Gestalt aufweisen.

Die Vitrektomienadel 1 ist im vorliegenden Beispiel mit dem Griffteil 8 verklebt, und insoweit mit diesem fest, insbesondere drehfest, verbunden.

Ausgehend vom Griffteil 8 weist die Vitrektomienadel 1 des Ausführungsbeispiels der FIG. 8 eine geraden Abschnitt 15 auf, der sich z.B. über etwa 2/3 der Länge der Vitrektomienadel 1 erstrecken kann. An den geraden Abschnitt 15 schließt sich eine Krümmung, konkret ein gekrümmter Abschnitt 27 an, der sich im Wesentlichen bis zum distalen Ende hin erstreckt. Der Durchbruch 14 liegt im vorliegenden Fall auf der Innenseite der Krümmung, d.h. der Durchbruch 14 ist auf der dem/den Krümmungsmittelpunkt/en zugewandten Seite ausgebildet.

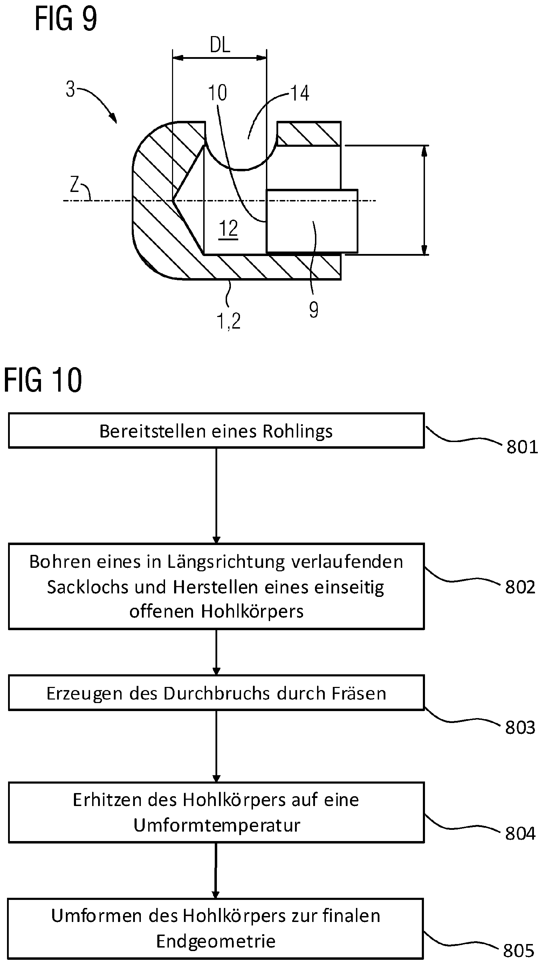

FIG. 9 zeigt einen vergrößerten Abschnitt des distalen Endes der Vitrektomienadel des Vitrektoms der FIG. 8. Die axiale innere Fläche des distalen Endes 3 weist im vorliegenden Beispiel eine etwa konische Form auf, die beispielsweise durch die Bohrgeometrie des zur Herstellung der Hohlnadel 2 verwendeten Bohrers gegeben sein kann. Die Bohrung ist im Wesentliche konzentrisch zur Zentralachse Z ausgebildet. Die Lichtaustrittsfläche 10 des Laserlichtleiters 9 ist in einem vorgegebenen Abstand DL von der axialen inneren Fläche des distalen Endes 3 beabstandet, wobei zwischen der Lichtaustrittsfläche 10 und der axialen inneren Fläche die Kavität 12 ausgebildet ist. Der Abstand DL kann beispielsweise 0,9 mm bis 1,2 mm betragen, wobei der Abstand DL vorzugsweise abhängig vom Außendurchmesser ist, und je kleiner ist, je kleiner der Außendurchmesser ist.

Im vorliegenden Beispiel ragt die Lichtaustrittsfläche 14 im Radialschnitt betrachtet über den, dem distalen Ende 3 abgewandten Rand des Durchbruchs 14 zum distalen Ende 3 hin hinaus. Der Laserlichtleiter 9 ist in der Hohlnadel 2 bzw. in der Vitrektomienadel 1 durch eine oder mehrere Klebestellen befestigt. Der Innendurchmesser der Hohlnadel 2 kann beispielsweise 0,45 mm betragen. Der Durchmesser des Laserlichtleiters 9 ist kleiner als der Innendurchmesser der Hohlnadel 2, so dass sich beispielsweise zwischen dem Laserlichtleiter 9 und der Innenwandung der Hohlnadel 2 ein Kanal ausbilden kann.

Im vorliegenden Beispiel ist der Durchbruch 14 durch einen senkrecht zur Zentralachse Z verlaufenden Schnitt oder Einschnitt gebildet, der beispielsweise durch ein senkrecht zur Zentralachse Z positioniertes bzw. orientiertes und bewegtes Fräswerkzeug hergestellt sein kann.

Im Zusammenhang mit FIG. 10 wird schließlich noch ein Verfahrensablauf zur Herstellung einer Vitrektomienadel im Rahmen der Erfindung beschrieben.

In einem ersten Verfahrensschritt 801 wir ein Rohling bereitgestellt, der eine langgestreckte Form aufweist, und massiv ausgebildet ist. Beispielsweise kann ein zylindrischer massiver Rohling verwendet werden. Als Material für den Rohling eignet sich Metall, vorzugsweise Titan oder eine Titanlegierung. Beispielsweise können Titanwerkstoff der Bezeichnung Grade 4 oder Grade 5 (nach ASTM-Standard) verwendet werden

In einem zweiten Verfahrensschritt 802 wird in den Rohling ein in dessen Längsrichtung verlaufendes Sackloch gebohrt, so dass ein Hohlkörper entsteht, der in Längsrichtung einseitig offen ist, d.h. der einseitig geschlossen ist.

In einem dritten Verfahrensschritt 803 wird der bezüglich der Längsrichtung radiale Durchbruch 14 an dem geschlossenen Ende des Sacklochs mittels eines Fräswerkzeugs erzeugt, derart, dass der Durchbruch 14 radial in das Sackloch mündet.

In einem vierten Verfahrensschritt 804 wird der Hohlkörper auf eine Umformungstemperatur erhitzt bzw. erwärmt, beispielsweise durch resistive Erwärmung mittels elektrischem Strom.

In einem fünften Verfahrensschritt 805 wird der Hohlkörper unter Verwendung geeigneter Umformwerkzeuge bzw. Gesenke zur gewünschten finalen Endgeometrie umgeformt.

In den Umgeformten Hohlkörper kann schließlich noch der Laserlichtleiter 9 eingebracht und fixiert werden. Ferner kann am proximalen Ende 6 eine Schraubverbindung oder eine anderweitige Verbindungsmöglichkeit mit einer Koppelfläche für den Laserlichtleiter 9 angebracht werden, so dass die Vitrektomienadel 1 mit einem wie z.B. in FIG. 6 ausgebildeten Griffteil 8 in zerstörungsfrei abnehmbarer Weise verbunden werden kann. Anstelle einer lösbaren Verbindung kann auch vorgesehen sein, dass die Hohlnadel 2 bzw. die Vitrektomienadel 1 fest mit dem Griffteil 8 verbunden, vorzugsweise verklebt, ist.

Mit dem Verfahren kann die hierin vorgeschlagene Vitrektomienadel vergleichsweise effizient und mit hoher Verfahrensstabilität und Wiederholbarkeit hergestellt werden. Ferner ist es durch die Wahl entsprechender Umformwerkzeuge möglich, unterschiedliche Formen und Geometrien für jeweils unterschiedliche Anwendungsfälle und Augengrößen bereitzustellen.

Insgesamt wird deutlich, dass die hierin vorgeschlagene Vitrektomienadel, das Vitrektom, die Vitrektomievorrichtung und das Verfahren zur Herstellung einer Vitrektomienadel die eingangs genannten Nachteile im Stand der Technik beseitigen.

### Bezugszeichenliste

- 1: Vitrektomienadel
- 2: Hohlnadel
- 3: distales Ende
- 4: Glaskörper
- 5: Auge
- 6: proximales Ende
- 7: Schraubverbinder
- 8: Griffteil
- 9: Laserlichtleiter
- 10: Lichtaustrittsfläche
- 11: nach innen gewandte Fläche
- 12: Kavität
- 13: Wandung
- 14: Durchbruch
- 15: gerader Abschnitt
- 15.1: weiterer gerader Abschnitt
- 16: Trokar
- 17: kontinuierliche Krümmung
- 18: Krümmung
- 19: Vitrektomievorrichtung
- 20: Zentraleinheit
- 21: Bedienkonsole
- 22: Laserlichtquelle
- 23: Aspirations- und Irrigationsvorrichtung
- 24: Steuereinheit
- 25: Vitrektom
- 26: Steuer- und/oder Signalleitung
- 27: gekrümmter Abschnitt

- 801 - 805: Verfahrensschritte
- A: Aktionsradius
- Da: Außendurchmesser
- Di: Innendurchmesser
- K: Krümmungsradius
- L: Länge
- M: Mittelachse
- R: radialer Mindestabstand
- Z: Zentralachse

## Patentansprüche

1. Vitrektomievorrichtung (19) mit einer Laserlichtquelle (22) und einer Vitrektomienadel (1) umfassend eine aus Metall, insbesondere Titan, hergestellten Hohlnadel (2) mit einem distalen Ende (3) zum Einführen in den Glaskörper (4) eines Auges (5) zur Durchführung einer Vitrektomie, und umfassend einen in der Hohlnadel (2) zu dem distalen Ende (3) geführten Laserlichtleiter (9) mit einer zum distalen Ende (3) hin orientierten Lichtaustrittsfläche (10), wobei im Bereich der Lichtaustrittsfläche (10) und der axial nach innen gewandeten Fläche (11) des distalen Endes (3) in der Hohlnadel (2) eine Kavität (12) ausgebildet ist und die Wandung (13) der Hohlnadel (2) im Bereich der Kavität (12) einen bezüglich der Mittelachse (M) der Hohlnadel (2) radial verlaufenden Durchbruch (14) aufweist, wobei die Vitrektomienadel (1), insbesondere die Hohlnadel (2), am proximalen Ende (6) einen geraden Abschnitt (15) aufweist, wobei die Mittelachse (M) im Bereich des geraden Abschnitts (15) eine Zentralachse (Z) definiert, und wobei die Mittelachse (M) der Hohlnadel (2) im Bereich des Durchbruchs (14) um einen vorgegebenen radialen Mindestabstand (R) von der Zentralachse (Z) beabstandet ist, wobei die Laserlichtquelle (22) ein Pulslaser ist und dazu eingerichtet ist, Laserpulse mit einer Pulsfrequenz von 40 Hz bis 60 Hz und einer Pulsenergie im Bereich von 0,5 bis 3 mJ zu erzeugen, so dass die im Inneren der Hohlnadel an der Lichtaustrittsfläche (10) des Laserlichtleiters (9) ausgehenden Laserpulse im Bereich der Kavität und des Durchbruchs Schockwellen erzeugen, die durch den Durchbruch (14) austreten und entsprechend das Glaskörpermaterial zertrümmern.

2. Vitrektomievorrichtung (19) nach Anspruch 1, wobei die Hohlnadel (2) ausgehend von dem geraden Abschnitt (15) zum distalen Ende (3) hin eine Krümmung (18) mit einem, vorzugsweise konstanten, Krümmungsradius (K) aufweist.

3. Vitrektomievorrichtung (1) nach Anspruch 2, wobei sich die Krümmung (18) ausgehend vom geraden Abschnitt (15) im Wesentlichen bis zum distalen Ende (3) hin erstreckt und/oder wobei es sich bei der Krümmung (18), ausgehend vom geraden Abschnitt zum distalen Ende (3) hin um eine kontinuierliche Krümmung (18) handelt.

4. Vitrektomievorrichtung (1) nach Anspruch 2, wobei die Hohlnadel (2) ausgehend von der Krümmung (18) zum distalen Ende (3) hin zumindest einen weiteren geraden Abschnitt (15.1) aufweist.

5. Vitrektomievorrichtung (1) nach einem der Ansprüche 1 bis 4, wobei die Hohlnadel (2) einen Außendurchmesser (Da) von 0,55 mm bis 0,65 mm, vorzugsweise 0,6 mm aufweist, und/oder wobei die Hohlnadel (2) einen Innendurchmesser (Di) von 0,35 mm bis 0,55 mm, insbesondere von 0,35 bis 0,45 mm, vorzugsweise von etwa 0,4 mm aufweist, und/oder wobei die Hohlnadel (2) eine längs der Mittelachse (M) gemessene Länge (L) von 18 mm bis 30 mm, vorzugsweise 20 mm bis 27 mm, weiter vorzugsweise etwa 25 mm, aufweist.

6. Vitrektomievorrichtung (1) nach einem der Ansprüche 1 bis 5, wobei der radiale Mindestabstand (R) im Bereich von mindestens 5 mm bis 30 mm liegt.

7. Vitrektomievorrichtung (1) nach einem der Ansprüche 1 bis 6, wobei der Durchbruch (14) an einer der Zentralachse (Z) zugewandten Seite der Hohlnadel (2) oder an einer der Zentralachse (Z) abgewandten Seite der Hohlnadel (2) ausgebildet ist.

8. Vitrektomievorrichtung nach einem der Ansprüche 1 bis 7, wobei die Vitrektomienadel (1) mit einem Handstück (8) verbunden ist, und das Handstück (8) und die Vitrektomienadel (1) ein Vitrektom (25) bilden.

9. Vitrektomievorrichtung (19) nach Anspruch 8, wobei die Vitrektomienadel (1) lösbar am Handstück (8) befestigt ist, insbesondere mittels eines am proximalen Ende (6) der Vitrektomienadel (1) ausgebildeten Schraubverbinders (7), oder wobei die Vitrektomienadel (1) fest mit dem Handstück (8) verbunden, insbesondere verklebt, ist.

10. Vitrektomievorrichtung (19) nach Anspruch 8 oder 9, umfassend eine Steuereinrichtung (24) zur Steuerung der Laserlichtquelle (22) und eine Anschlussschnittstelle zum Anschließen des Vitrektoms (25), wobei die Steuereinrichtung (24) dazu eingerichtet ist, bei angeschlossenem Vitrektom (25) zumindest die Laserlichtquelle (22) zur Ausführung einer Vitrektomie anzusteuern.

11. Vitrektomievorrichtung nach einem der Ansprüche 1 bis 10, wobei die Laserlichtquelle (22) ein Nd:YAG Pulslaser ist, und/oder wobei der Laserlichtleiter (9) einen Außendurchmesser von 250 µm bis 300 µm aufweist.

12. Verfahren zur Herstellung einer Vitrektomievorrichtung (19) nach einem der Ansprüche 1 bis 11, umfassend:
Herstellen der Vitrektomienadel (1) durch:
- Bereitstellen (801) eines langgestreckten massiven, insbesondere zylindrischen, Rohlings aus Metall, insbesondere Titan,
- Erzeugen (802) eines Hohlkörpers aus dem Rohling durch Bohren eines zentralen, in Längsrichtung des Rohlings verlaufenden Sacklochs mittels eines Bohrwerkzeugs;
- Erzeugen (803) des bezüglich der Längsrichtung radialen Durchbruchs an dem geschlossenen Ende des Sacklochs mittels eines Fräswerkzeugs, derart, dass der Durchbruch radial in das Sackloch mündet;
- Erwärmen (804) des Hohlkörpers auf eine Umformungstemperatur, insbesondere im Bereich zwischen 60°C und 140°C, insbesondere zwischen 100°C und 130°C, weiter insbesondere bei ungefähr 120°C; und
- Umformen (805) des Hohlkörpers auf die finale Endgeometrie der Vitrektomienadel mittels eines Umformwerkzeugs;
Bereitstellen einer Laserlichtquelle (22), welche ein Pulslaser ist und dazu eingerichtet ist, Laserpulse mit einer Pulsfrequenz von 40 Hz bis 60 Hz und einer Pulsenergie im Bereich von 0,5 bis 3 mJ zu erzeugen; und
Platzieren eines Laserlichtleiters (9) in dem Hohlkörper derart, dass im Betrieb durch die Laserlichtquelle (22) die im Inneren der Hohlnadel an der Lichtaustrittsfläche (10) des Laserlichtleiters (9) ausgehenden Laserpulse im Bereich der Kavität und des Durchbruchs Schockwellen erzeugen, die durch den Durchbruch (14) austreten und entsprechend das Glaskörpermaterial zertrümmern.

13. Verfahren nach Anspruch 12, wobei der Rohling einen Außendurchmesser von 0,2 mm bis 1 mm aufweist.

14. Verfahren nach einem der Ansprüche 12 und 13, wobei das Erwärmen eine resistive elektrische Erwärmung mittels elektrischem Strom und/oder eine induktive Erwärmung umfasst.

15. Verfahren nach einem der Ansprüche 12 bis 14, wobei der Laserlichtleiter (9) einen Durchmesser von 250 µm bis 300 µm, insbesondere von etwa 270 µm, aufweist.

## Claims

1. Vitrectomy device (19) with a laser light source (22) and a vitrectomy needle (1) comprising a hollow needle (2) made of metal, in particular titanium, with a distal end (3) for insertion into the vitreous body (4) of an eye (5) for performing a vitrectomy, and comprising a laser light guide (9) guided in the hollow needle (2) to the distal end (3) with a light exit surface (10) oriented towards the distal end (3), wherein a cavity (12) is formed in the hollow needle (2) in the region of the light-emitting surface (10) and the axially inwardly facing surface (11) of the distal end (3) and the wall (13) of the hollow needle (2) in the region of the cavity (12) has an aperture (14) extending radially with respect to the central axis (M) of the hollow needle (2), wherein the vitrectomy needle (1), in particular the hollow needle (2), has a straight section (15) at the proximal end (6), wherein the central axis (M) defines a central axis (Z) in the region of the straight section (15), and wherein the central axis (M) of the hollow needle (2) is spaced apart from the central axis (Z) by a predetermined minimum radial distance (R) in the region of the aperture (14), wherein the laser light source (22) is a pulse laser and is set up to generate laser pulses with a pulse frequency of 40 Hz to 60 Hz and a pulse energy in the range from 0.5 to 3 mJ, so that the laser pulses emitted in the interior of the hollow needle at the light exit surface (10) of the laser light guide (9) generate shock waves in the region of the cavity and the aperture which exit through the aperture (14) and accordingly shatter the glass body material.

2. Vitrectomy device (19) according to claim 1, wherein the hollow needle (2), starting from the straight section (15) towards the distal end (3), has a curvature (18) with a, preferably constant, radius of curvature (K).

3. The vitrectomy device (1) according to claim 2, wherein the curvature (18) extends from the straight portion (15) substantially to the distal end (3) and/or wherein the curvature (18) is a continuous curvature (18) from the straight portion to the distal end (3).

4. Vitrectomy device (1) according to claim 2, wherein the hollow needle (2) has at least one further straight section (15.1) starting from the curvature (18) towards the distal end (3).

5. Vitrectomy device (1) according to one of claims 1 to 4, wherein the hollow needle (2) has an outer diameter (Da) of 0.55 mm to 0.65 mm, preferably 0.6 mm, and/or wherein the hollow needle (2) has an inner diameter (Di) of 0.35 mm to 0.55 mm, in particular of 0.35 to 0.45 mm, preferably of about 0.4 mm, and/or wherein the hollow needle (2) has a length (L) measured along the center axis (M) of 18 mm to 30 mm, preferably 20 mm to 27 mm, more preferably about 25 mm.

6. The vitrectomy device (1) according to any one of claims 1 to 5, wherein the minimum radial distance (R) is in the range of at least 5 mm to 30 mm.

7. Vitrectomy device (1) according to one of claims 1 to 6, wherein the aperture (14) is formed on a side of the hollow needle (2) facing the central axis (Z) or on a side of the hollow needle (2) facing away from the central axis (Z).

8. The vitrectomy device according to any one of claims 1 to 7, wherein the vitrectomy needle (1) is connected to a handpiece (8), and the handpiece (8) and the vitrectomy needle (1) form a vitrectome (25).

9. Vitrectomy device (19) according to claim 8, wherein the vitrectomy needle (1) is detachably attached to the handpiece (8), in particular by means of a screw connector (7) formed at the proximal end (6) of the vitrectomy needle (1), or wherein the vitrectomy needle (1) is firmly connected, in particular glued, to the handpiece (8).

10. Vitrectomy device (19) according to claim 8 or 9, comprising a control device (24) for controlling the laser light source (22) and a connection interface for connecting the vitrectome (25), wherein the control device (24) is set up to control at least the laser light source (22) for performing a vitrectomy when the vitrectome (25) is connected.

11. The vitrectomy device according to any one of claims 1 to 10, wherein the laser light source (22) is a Nd:YAG pulsed laser, and/or wherein the laser light guide (9) has an outer diameter of 250 µm to 300 µm.

12. A method of manufacturing a vitrectomy device (19) according to any one of claims 1 to 11, comprising:
Manufacture the vitrectomy needle (1):
- Providing (801) an elongated solid, in particular cylindrical, blank made of metal, in particular titanium,
- Generating (802) a hollow body from the blank by drilling a central blind hole extending in the longitudinal direction of the blank by means of a drilling tool;
- Generating (803) the radial aperture at the closed end of the blind hole with respect to the longitudinal direction by means of a milling tool in such a way that the opening opens radially into the blind hole;
- Heating (804) the hollow body to a forming temperature, in particular in the range between 60°C and 140°C, in particular between 100°C and 130°C, further in particular at approximately 120°C; and
- Forming (805) the hollow body to the final end geometry of the vitrectomy needle using a forming tool;
Providing a laser light source (22) which is a pulsed laser and is adapted to generate laser pulses with a pulse frequency of 40 Hz to 60 Hz and a pulse energy in the range of 0.5 to 3 mJ; and
Placing a laser light guide (9) in the hollow body in such a way that, during operation, the laser pulses emitted by the laser light source (22) inside the hollow needle at the light exit surface (10) of the laser light guide (9) generate shock waves in the region of the cavity and the aperture, which exit through the aperture (14) and accordingly shatter the glass body material.

13. Method according to claim 12, wherein the blank has an outer diameter of 0.2 mm to 1 mm.

14. Method according to any one of claims 12 and 13, wherein the heating comprises resistive electrical heating by means of electric current and/or inductive heating.

15. Method according to any one of claims 12 to 14, wherein the laser light guide (9) has a diameter of 250 µm to 300 µm, in particular of about 270 µm.

## Revendications

1. Dispositif de vitrectomie (19) avec une source de lumière laser (22) et une aiguille de vitrectomie (1) comprenant une aiguille creuse (2) fabriquée en métal, en particulier en titane, avec une extrémité distale (3) pour l'introduction dans le corps vitré (4) d'un oeil (5) pour la réalisation d'une vitrectomie, et comprenant un guide de lumière laser (9) guidé dans l'aiguille creuse (2) vers l'extrémité distale (3) avec une surface de sortie de lumière (10) orientée vers l'extrémité distale (3), une cavité (12) étant formée dans l'aiguille creuse (2) dans la zone de la surface de sortie de lumière (10) et de la surface (11) de l'extrémité distale (3) qui est tournée axialement vers l'intérieur, et la paroi (13) de l'aiguille creuse (2) présentant dans la zone de la cavité (12) und percée (14) qui s'étend radialement par rapport à l'axe médian (M) de l'aiguille creuse (2), l'aiguille de vitrectomie (1), en particulier l'aiguille creuse (2), présente à l'extrémité proximale (6) une section rectiligne (15), l'axe central (M) définissant un axe central (Z) dans la zone de la section rectiligne (15), et l'axe central (M) de l'aiguille creuse (2) étant espacé de l'axe central (Z) dans la zone de la percée (14) d'une distance radiale minimale (R) prédéfinie, la source de lumière laser (22) étant un laser à impulsions et étant conçue à cet effet, générer des impulsions laser avec une fréquence d'impulsion de 40 Hz à 60 Hz et une énergie d'impulsion dans la plage de 0,5 à 3 mJ, de sorte que les impulsions laser partant à l'intérieur de l'aiguille creuse au niveau de la surface de sortie de lumière (10) du guide de lumière laser (9) génèrent dans la zone de la cavité et de la percée des ondes de choc qui sortent par la percée (14) et brisent en conséquence le matériau du corps en verre.

2. Dispositif de vitrectomie (19) selon la revendication 1, dans lequel l'aiguille creuse (2) présente, en partant de la section rectiligne (15) vers l'extrémité distale (3), une courbure (18) avec un rayon de courbure (K), de préférence constant.

3. Dispositif de vitrectomie (1) selon la revendication 2, dans lequel la courbure (18) s'étend depuis la partie rectiligne (15) essentiellement jusqu'à l'extrémité distale (3) et/ou dans lequel la courbure (18) est une courbure continue (18) depuis la partie rectiligne jusqu'à l'extrémité distale (3).

4. Dispositif de vitrectomie (1) selon la revendication 2, dans lequel l'aiguille creuse (2) présente au moins une autre section rectiligne (15.1) en partant de la courbure (18) vers l'extrémité distale (3).

5. Dispositif de vitrectomie (1) selon l'une des revendications 1 à 4, dans lequel l'aiguille creuse (2) présente un diamètre extérieur (Da) de 0,55 mm à 0,65 mm, de préférence de 0,6 mm, et/ou dans lequel l'aiguille creuse (2) présente un diamètre intérieur (Di) de 0,35 mm à 0,55 mm, en particulier de 0,35 à 0,45 mm, de préférence d'environ 0,4 mm, et/ou dans laquelle l'aiguille creuse (2) présente une longueur (L), mesurée le long de l'axe central (M), de 18 mm à 30 mm, de préférence de 20 mm à 27 mm, de préférence encore d'environ 25 mm.

6. Dispositif de vitrectomie (1) selon l'une quelconque des revendications 1 à 5, dans lequel la distance radiale minimale (R) est comprise entre au moins 5 mm et 30 mm.

7. Dispositif de vitrectomie (1) selon l'une des revendications 1 à 6, dans lequel la percée (14) est formé sur un côté de l'aiguille creuse (2) tourné vers l'axe central (Z) ou sur un côté de l'aiguille creuse (2) opposé à l'axe central (Z).

8. Dispositif de vitrectomie selon l'une quelconque des revendications 1 à 7, dans lequel l'aiguille de vitrectomie (1) est reliée à une pièce à main (8), et la pièce à main (8) et l'aiguille de vitrectomie (1) forment un vitrectome (25).

9. Dispositif de vitrectomie (19) selon la revendication 8, dans lequel l'aiguille de vitrectomie (1) est fixée de manière amovible à la pièce à main (8), notamment au moyen d'un connecteur à vis (7) formé à l'extrémité proximale (6) de l'aiguille de vitrectomie (1), ou dans lequel l'aiguille de vitrectomie (1) est reliée de manière fixe, notamment par collage, à la pièce à main (8).

10. Dispositif de vitrectomie (19) selon la revendication 8 ou 9, comprenant un dispositif de commande (24) pour commander la source de lumière laser (22) et une interface de connexion pour connecter le vitrectome (25), dans lequel le dispositif de commande (24) est adapté pour commander au moins la source de lumière laser (22) pour effectuer une vitrectomie lorsque le vitrectome (25) est connecté.

11. Dispositif de vitrectomie selon l'une des revendications 1 à 10, dans lequel la source de lumière laser (22) est un laser pulsé Nd:YAG, et/ou dans lequel le guide de lumière laser (9) présente un diamètre extérieur de 250 µm à 300 µm.

12. Procédé de fabrication d'un dispositif de vitrectomie (19) selon l'une quelconque des revendications 1 à 11, comprenant:
Fabrication de l'aiguille de vitrectomie (1) par:
- Mettre à disposition (801) d'une ébauche massive allongée, en particulier cylindrique, en métal, en particulier en titane,
- produire (802) un corps creux à partir de l'ébauche en perçant un trou borgne central s'étendant dans la direction longitudinale de l'ébauche au moyen d'un outil de perçage;
- réaliser (803) la percée radiale par rapport à la direction longitudinale à l'extrémité fermée du trou borgne au moyen d'un outil de fraisage, de telle sorte que la percée débouche radialement dans le trou borgne;
- chauffer (804) le corps creux à une température de formage, en particulier dans la plage comprise entre 60°C et 140°C, en particulier entre 100°C et 130°C, plus particulièrement à environ 120°C; et
- formage (805) du corps creux à la géométrie finale de l'aiguille de vitrectomie au moyen d'un outil de formage;
Mettre à disposition une source de lumière laser (22) qui est un laser à impulsions et qui est adaptée pour générer des impulsions laser ayant une fréquence d'impulsion de 40 Hz à 60 Hz et une énergie d'impulsion dans la plage de 0,5 à 3 mJ; et
placer une fibre optique laser (9) dans le corps creux de telle sorte qu'en fonctionnement, grâce à la source de lumière laser (22), les impulsions laser émises à l'intérieur de l'aiguille creuse au niveau de la surface de sortie de lumière (10) de la fibre optique laser (9) génèrent dans la zone de la cavité et de la percée des ondes de choc qui sortent par la percée (14) et brisent de manière correspondante le matériau du corps en verre.

13. Procédé selon la revendication 12, dans lequel l'ébauche présente un diamètre extérieur de 0,2 mm à 1 mm.

14. Procédé selon l'une des revendications 12 et 13, dans lequel le chauffage comprend un chauffage électrique résistif au moyen d'un courant électrique et/ou un chauffage inductif.

15. Procédé selon l'une des revendications 12 à 14, dans lequel le guide de lumière laser (9) présente un diamètre de 250 µm à 300 µm, en particulier d'environ 270 µm.
